# EUROPEAN PATENT SPECIFICATION

(11) **EP 0 551 588 B1**
(45) Date of publication and mention of the grant of the patent: **19.03.1997**
(21) Application number: 92119591.3
(22) Date of filing: 17.11.1992
(51) Int. Cl.: A61B 17/74

(54) **Intramedullary nail for treating fractures of the proximal femur**
Verriegelungsnagel für die Versorgung von Frakturen des proximalen Femurs
Clou intermédullaire pour la traitement des fractures du fémur proximal

(30) Priority: 14.01.1992 DE 9200328 U
(43) Date of publication of application: 21.07.1993
(73) Proprietor: Howmedica GmbH, D-24232 Schönkirchen (DE)
(72) Inventor: Harder, Hans Erich, W-2316 Probsteierhagen (DE); Grosse, Arsène Jérome, Dr., F-67000 Strassbourg (FR)
(74) Representative: Patentanwälte Hauck, Graalfs, Wehnert, Döring, Siemons

(56) References cited:
- FR-A- 2 559 376
- GB-A- 2 090 745
- GB-A- 2 209 947
- US-A- 4 733 654
- US-A- 4 827 917

## Description

The present invention relates to a locking nail for treating fractures of the proximal femur according to the preamble of claim 1.

EP 0257118 discloses a locking nail for treating subtrochantic fractures. A nail shaft proximally inserted into the marrow space of the femur is provided with an oblique bore for receiving the femur neck screw. The locking nail is distally provided with a pair of cross-bores to receive bone screws. The femur neck screw is freely axially movable in the cross-bore and the locking nail is provided with a securing means selectively preventing a turning of the femur neck screw but allowing an axial displacement. For an osteosynthesis supplement of such type the locking nail is not used per se for treating the fracture, but is provided to guide and hold the femur neck screw.

A locking nail of the kind indicated above has become known from the US-A-4 827 917, wherein the proximal end of the locking nail has two parallel oblique bores for the receipt of neck screws. The diameter of the more proximally located neck screw is smaller than that of the other neck screw. The neck screws are fittingly accommodated by the oblique bores of the locking nail.

The locking nails used for the known osteosynthesis aids are designed to be relatively long so that the femur is hurt over almost its total length when being driven in. When a pair of femur neck screws is used, they both are located within a plane which does not always fit to the conditions to be met, in particular in case of subcapital fractures.

Therefore, it is the object of the present invention to provide a locking nail which is particularly suited for treating subcapital fractures.

According to the invention the object is solved by the features of claim 1.

According to the invention the proximally located oblique bore of the nail shaft has a larger diameter than the respective femur neck screw to allow different angular orientations. Preferrably, the proximal bore in cross section has a larger diameter along an axis perpendicular to the nail axis, e.g. the bore may be oval in cross section. In this manner, the associated femur neck screw inserted may pivot in the oblique bore to some limited extent to be oriented in a desired way. In particular, the neck screw may be positioned in an angular position substantially different with respect to the position of the other femur neck screw. This is of a particular advantage in treating subcapital fractures.

According to a further embodiment of the invention the nail shaft is shaped to be relatively short. The proximal portion of the nail shaft is formed substantially cylindrical, while conically tapering towards the distal end in the distal area. The length of the nail shaft between the distal end and the distally located oblique bore maximally is 1.5 times the length of the nail shaft between the proximal end and the distally located oblique bore. Therefore, the femur is minimally hurt only when implanting the nail shaft.

According to the invention the proximal portion of the nail shaft has a relatively large diameter. There is an advantage, therefore, when the nail shaft is curved in the lateral medial plane to fit to the natural curvature of the femur.

According to a still further embodiment of the invention the nail shaft is axially provided with a longitudinal bore. Thus the nail shaft may be driven in by means of a guide lance.

Since the nail shaft is relatively short, a further embodiment of the invention provides for a single cross-bore in the distal end portion to lock the nail in the femur.

By way of an example, the invention will now be described with reference to the accompanying drawing.
- Fig. 1: is a side view of a nail shaft of the locking nail according to the invention,
- Fig. 2: is a section through the locking nail portion of Fig. 1 along the line 2-2,
- Fig. 3: is a side view of a femur neck screw for the nail shaft of Figs. 1 or 2,
- Fig. 4: is a side view of an alternative femur neck screw for the nail of Figs. 1 and 2, and
- Fig. 5: is a section through the femur neck screw of Fig. 4 along the line 5-5.

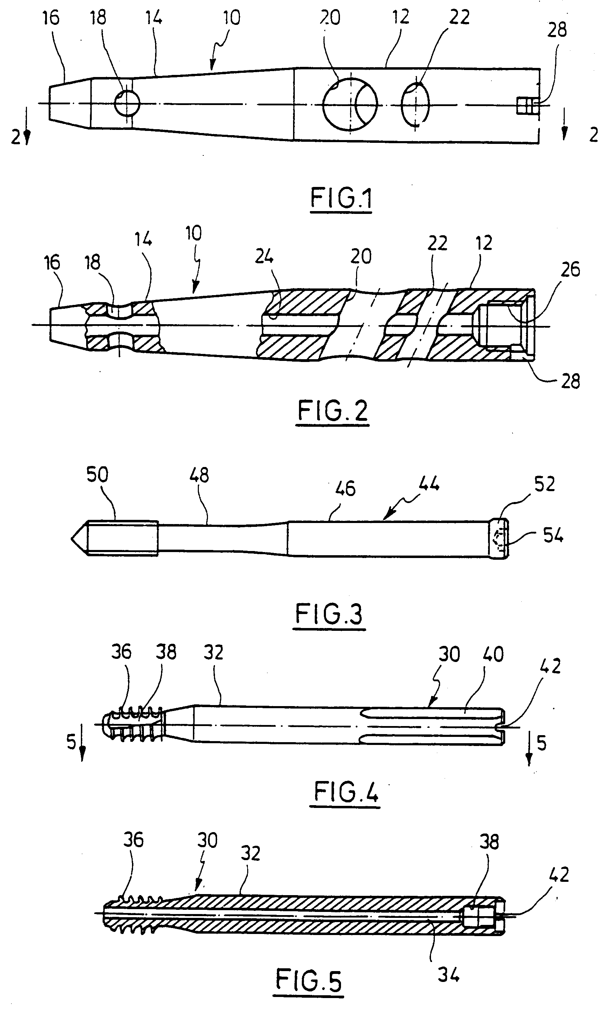

A nail shaft 10 forming part of a locking nail comprises a cylindrical portion 12 in the proximal area and a substantially conical portion 14 in the distal area. The total length is 120 mm for example, wherein both the portions 12, 14 are substantially of equal length. As may be seen a truncated tip 16 is formed at the first tapered portion of the nail portion 14. The distal end portion is provided with a cross- bore 18 for receiving a bone screw not shown. In the proximal portion, or respectively the section 12 a pair of oblique or inclined bores 20 and 22 is provided for receiving femur neck screws shown in Figs. 3 to 5. The angle of the oblique bores 20, 22 which is equal for both bores may be between 25° and 40° relative to a perpendicular on the longitudinal axis of the nail shaft 10.

As Fig. 2 shows, the nail shaft 10 is curved in the lateral medial plane corresponding to the natural curvature of the human femur. The nail shaft 10 further includes a longitudinal bore 24 extending in correspondence with the curvature of the nail (Fig. 2). At the proximal end the longitudinal bore is extended to a threaded bore 26. For example it serves to receive a suited positioning device (not shown). Furthermore, the proximal end includes a recess 28 which is suited to orient the positioning device to be applied as well to rotate the nail in a desired rotational position when driving in.

The oblique bore 20 having a larger diameter receives a femur neck screw 30 as shown in Figs. 4 and 5. The illustration of the femur neck screw in Figs. 3 to 5 is not on scale. Accordingly, the femur neck screw 30 has a diameter which is minimally smaller only than the diameter of the oblique bore 20. The femur neck screw 30 includes a cylindrical shaft 32 having an axial bore 34. At the front end a thread 36 is formed which is self-cutting by a cutting groove 38. At the rearward region a plurality of circumferentially spaced grooves 40 is formed which extend parallel to the axis. The grooves may receive the free end of a screw (not shown) which is turned into the threaded bore 26 to fix the femur neck screw 30 in a rotational position, but allows an axial displacement as disclosed in the European Patent 257118 referred to above. A fixing of the femur neck screw 30 of this type is accomplished only then, however, when there is no femur neck screw inserted through the second oblique bore 22. The femur neck screw 30 has an end portion including an inner threaded bore 38 for the mount of a suitable instrument. Four recesses 42 equally spaced peripherally allow to apply a turning force.

A femur neck screw 44 (Fig. 3) is inserted through the oblique bore 22 which screw has a cylindrical shaft portion 46 extending into a tapered shaft portion 48 towards the end at which a threaded portion 50 is formed. The head 52 of the femur neck screw 44 comprises an inner hexagonal recess 54.

Fig. 1 shows that the oblique bore 22 is not circular, but oval. When the diameter of the cylindrical shaft portion 46 is 6.5 mm for example, the large diameter of the oblique bore 22 amounts to 10 mm. As may be seen, the large diameter of the cross-sectional area of the bore 22 extends oblique with respect to the longitudinal axis of the nail shaft 10. Therefore, the femur neck screw 44 may be oriented with respect to the oblique bore 22 in accordance with the conditions to be met.

## Claims

1. A locking nail for treating subcapital fractures, comprising neck screws (30, 44) and a nail shaft (10) including a cross-bore (18) for receiving a bone screw in its distal end portion (14) and a pair of oblique bores (20, 22) for receiving femur neck screws (30, 44) in its proximal portion (12), the oblique bores (20, 22) having an axis inclined relative to the longitudinal and a transverse axis of the nail shaft (10), and being spaced along the longitudinal axis of the nail shaft (10), at least one (44) of the neck screws for the proximally located oblique bore having a cylindrical portion (46), characterized in that the proximally located oblique bore (22) has a larger diameter than the cylindrical portion (46) of the associated femur neck screw (44) such that it can be oriented in different angular positions.

2. The locking nail of claim 1, characterized in that the cross section of the proximal oblique bore (22) is oval.

3. The locking nail of claim 1 or 2, characterized in that the length of the nail shaft (10) between the distal end and the distally located oblique bore (20) maximally is 1.5 times the length of the nail shaft (10) between the proximal end and the distally located oblique bore (20).

4. The locking nail of claims 1 to 3, characterized in that the nail shaft (10) is formed substantially cylindrical (12) in its proximal portion and conically (14) in its distal portion.

5. The locking nail of one of claims 1 to 4, characterized in that the nail shaft (10) is curved in the lateral medial plane to suit the natural curvature of the femur.

6. The locking nail of one of claims 1 to 5, characterized in that the nail shaft (10) comprises an axial longitudinal bore (24).

7. The locking nail of one of claims 1 to 6, characterized in that a single cross bore (18) is provided in the distal end portion.

## Patentansprüche

1. Ein Verriegelungsnagel für die Versorgung von subkapitalen Frakturen mit Halsschrauben (30, 40) und einem Nagelschalt (10), der im distalen Endbereich (14) eine Querdurchbohrung (18) zur Aufnahme einer Knochenschraube aufweist und im proximalen Bereich (12) zwei Schrägdurchbohrungen (20, 22), die eine relativ zur Längs- und Querachse des Nagelschafts (10) sich neigende Achse haben und entlang der Längsachse des Nagelschafts (10) beabstandet sind, wobei wenigstens eine (44) der Halsschrauben für die proximal gelegene Schrägdurchbohrung einen zylindrischen Abschnitt (46) aufweist, dadurch gekennzeichnet, daß die proximal angeordnete Schrägdurchbohrung (22) einen größeren Durchmesser als der zylindrische Abschnitt (46) der zugehörigen Schenkelhalsschraube (44) hat, so daß sie in verschiedenen Winkelpositionen orientiert sein kann.

2. Verriegelungsnagel nach Anspruch 1, dadurch gekennzeichnet, daß der Querschnitt der proximalen Schrägdurchbohrung oval ist.

3. Verriegelungsnagel nach Anspruch 1 oder 2, dadurch gekennzeichnet, daß die Länge des Nagelschafts (10) zwischen dem distalen Ende und der distal gelegenen Schrägdurchbohrung (20) maximal das 1,5 fache der Länge des Nagelschafts (10) zwischen dem proximalen Ende und der distal gelegenen Schrägdurchbohrung (20) beträgt.

4. Verriegelungsnagel nach einem der Ansprüche 1 bis 3, dadurch gekennzeichnet, daß der Nagelschaft (10) in seinem proximalen Abschnitt im wesentlichen zylindrisch (12) und in seinem distalen Abschnitt konisch (14) geformt ist.

5. Verriegelungsnagel nach einem der Ansprüche 1 bis 4, dadurch gekennzeichnet, daß der Nagelschaft (10) in der lateral-medialen Ebene in Anpassung an die natürliche Krümmung des Femurs gekrümmt ist.

6. Verriegelungsnagel nach einem der Ansprüche 1 bis 5, dadurch gekennzeichnet, daß der Nagelschaft (10) eine axiale Längsdurchbohrung (24) aufweist.

7. Verriegelungsnagel nach einem der Ansprüche 1 bis 6, dadurch gekennzeichnet, daß eine einzelne Querdurchbohrung (18) im distalen Endabschnitt vorgesehen ist.

## Revendications

1. Clou de blocage pour le traitement de fractures sous-capitales, comportant des vis pour col (30, 44) et une tige (10) de clou présentant une lumière transversale (18) destinée à recevoir une vis pour os dans sa partie extrême distale (14), et deux lumières obliques (20, 22) destinées à recevoir des vis (30, 44) pour col de fémur dans sa partie proximale (12), les lumières obliques (20, 22) ayant un axe incliné par rapport à l'axe longitudinal et à un axe transversal de la tige (10) du clou, et étant espacées le long de l'axe longitudinal de la tige (10) du clou, au moins l'une (44) des vis pour col, destinée à la lumière oblique située du côté proximal, ayant une partie cylindrique (46), caractérisé en ce que la lumière oblique (22) située du côté proximal présente un plus grand diamètre que la partie cylindrique (46) de la vis associée (44) pour col de fémur afin qu'elle puisse être orientée dans des positions angulaires différentes.

2. Clou de blocage selon la revendication 1, caractérisé en ce que la section transversale de la lumière oblique proximale (22) est ovale.

3. Clou de blocage selon la revendication 1 ou 2, caractérisé en ce que la longueur de la tige (10) du clou entre l'extrémité distale et la lumière oblique (20) située du côté distal est égale au maximum à 1,5 fois la longueur de la tige (10) du clou comprise entre l'extrémité proximale et la lumière oblique (20) située du côté distal.

4. Clou de blocage selon les revendications 1 à 3, caractérisé en ce que la tige (10) du clou est formée de façon à être sensiblement cylindrique (12) dans sa partie proximale et conique (14) dans sa partie distale.

5. Clou de blocage selon l'une des revendications 1 à 4, caractérisé en ce que la tige (10) du clou est courbée dans le plan latéral interne pour s'adapter à la courbure naturelle du fémur.

6. Clou de blocage selon l'une des revendications 1 à 5, caractérisé en ce que la tige (10) du clou présente une lumière longitudinale axiale (24).

7. Clou de blocage selon l'une des revendications 1 à 6, caractérisé en ce qu'une lumière transversale unique (18) est prévue dans la partie extrême distale.
